# EUROPÄISCHE PATENTSCHRIFT

(11) **EP 1 329 202 B1**
(45) Veröffentlichungstag und Bekanntmachung des Hinweises auf die Patenterteilung: **10.03.2004**
(21) Anmeldenummer: 02001038.5
(22) Anmeldetag: 18.01.2002
(51) Int. Cl.: A61B 19/00, A61B 6/12

(54) **Verfahren und Einrichtung zur Zuordnung einer digitalen Bildinformation zu den Navigationsdaten eines medizinischen Navigationssystems**
Method and device for allocating digital image information to navigation data of a medical navigation system
Méthode et dispositif d'allocation d'information d'image numérique aux données de navigation d'un système de navigation médicale

(43) Veröffentlichungstag der Anmeldung: 23.07.2003
(73) Patentinhaber: BrainLAB AG, 85551 Kirchheim/Heimstetten (DE); Instrumentarium Imaging Ziehm GmbH, 90451 Nürnberg (DE)
(72) Erfinder: Wist, Henrik, 80538 München (DE); Ritter, Alf Dr., 85253 Erdweg (DE); Zeiss, Mario, 85586 Poing (DE); Rotermund-Buwen, Hanna, 90513 Zirndorf (DE); Lang, Helmar Werner, 90513 Zirndorf (DE); Strobel, Jorg Peter Dr., 91301 Forchheim (DE); Kreuzer, Klaus-Peter, 90768 Furth (DE)
(74) Vertreter: Schwabe - Sandmair - Marx

(56) Entgegenhaltungen:
- WO-A-00/56215
- US-A1- 2001 036 245

## Beschreibung

Die vorliegende Erfindung betrifft die Zuordnung einer digitalen Bildinformation zu den Navigationsdaten eines medizinischen Navigationssystems. Insbesondere findet die Erfindung ihre Anwendung im Bereich der apparativen Unterstützung unter anderem von orthopädischen Eingriffen, der sogenannten bildunterstützten Chirurgie (image guided surgery).

Gerade bei Eingriffen an der Wirbelsäule eines Patienten ist es von besonderer Wichtigkeit, dass mit hoher Genauigkeit operiert wird, und um den Arzt hierbei zu unterstützen, wird immer öfter mit medizinischen Navigationssystemen gearbeitet, die ein Patienten- und Instrumententracking gestatten und visuelle Hilfe bei der Vornahme von Operationstätigkeiten bieten. Ein solches medizinisches Navigationssystem ist beispielsweise aus der DE 196 39 615 C2 bekannt. Um eine möglichst optimale visuelle Unterstützung bereitstellen zu können, können zur Ergänzung der Bilder aus dem Navigationssystem, welche aus vorab erstellten Schichtbilderfassungen (CT, MR, PET) stammen, noch zusätzlich unter anderem Röntgenbilder oder Bildinformationen des Patienten mit in die dem Arzt bereitgestellten Informationen eingespielt werden, da solche Röntgenbilder sehr genaue Abbildungen der Knochenstrukturen gestatten. Die Röntgenbilder werden meist mit fahrbaren C-Bogen-Röntgengeräten erstellt, und dabei ist es wichtig, eine genaue Zuordnung des erstellten Röntgenbildes zu den vom Navigationssystem bereitgestellten Positions- und Bilddaten für den Patientenkörperteil sicherzustellen, damit die Information aus dem Röntgenbild auf dem Bildschirm des Navigationsgerätes auch im richtigen Positionsverhältnis dargestellt wird. Die US-Patente US 5,799,055, US 3,577,160, US 5,788,431, US 5,967,982 und US 5,772,594 beschäftigen sich mit einer solchen Röntgenbildregistrierung im Rahmen chirurgischer Navigationsverfahren.

Bei all diesen Verfahren wird noch eine analoge Technik eingesetzt, die mit Hilfe eines analogen Videosignal-Ausgangs am C-Bogen, BNC-Kabeln und einem Videosignal-Eingang am Computersystem arbeitet. Dabei liegt kontinuierlich die Bildinformation des C-Bogens an dem Videosignal-Eingang des Navigationssystems an, ebenso wie die Tracking-Informationen des medizinischen Navigationssystems ständig aktuell zur Verfügung gestellt werden. Das Computersystem des Navigationssystems verarbeitet dann die Videodaten und "registriert" sie, d. h. das System bestimmt die genaue Position von Bilddaten in Relation zu einer vorher definierten Referenz, zum Beispiel am Patienten. Es ist danach möglich, auf der Basis dieser gewonnenen Bilddaten die Position eines Instrumentes in "Echtzeit" auch gegenüber der vom Röntgenbild her dargestellten Knochenstruktur darzustellen, d. h. eine Navigationshilfe für den Arzt bereitzustellen.

Die Registrierung wird bei den derzeit bekannten analogen Systemen entweder manuell oder mit Hilfe komplexer Röntgensensoren gestartet. Dabei berücksichtigt das manuelle Verfahren nicht die zwischen der Bilderfassung am C-Bogen und der Bilderfassung am Navigationssystem mögliche Patientenbewegung, und das sensorische Verfahren beinhaltet eine sehr hohe Fehlerrate aufgrund der zusätzlichen Sensoren. Aus der WO 00/56215 ist ein digitales System bekannt, das ebenfalls Sensoren verwendet, um den Zeitpunkt der Bildaufnahme zu bestimmen. Dieses Dokument offenbart die Merkmale der Oberbegriffe der Ansprüche 1 und 13.

Auf dem Markt werden derzeit immer häufiger digital arbeitende C-Bogen-Röntgengeräte vorgestellt, die Bilder mit höherer Auflösung liefern können, und auch die Möglichkeit haben, mehrere Bilder zu speichern. Der Einsatz solcher digitaler C-Bogen-Röntgengeräte, die eine digitale Bildinformation liefern, ist auch im Rahmen der Navigation unter Zuhilfenahme registrierter Röntgenbilder wünschenswert, jedoch bestehen hierbei noch technische Umsetzungsprobleme, die damit zusammenhängen, dass nach dem Erstellen des digitalen Röntgenbildes eine Verarbeitungszeit im C-Bogen auftritt, und insgesamt auch eine gewisse Zeit zur Übermittlung von Signalen vom C-Bogen zum Navigationssystem benötigt wird. Es kann also eine gewisse Zeit vergehen, bis das Navigationssystem ein verarbeitetes Bild erhält und dann die zugehörige Patientenposition im Navigationssystem erfassen kann. In dieser Zeit kann sich der Patient oder aber der C-Bogen bewegt haben, selbst wenn diese Bewegung nur durch die Atemtätigkeit erfolgt. Fehlregistrierungen sind deshalb grundsätzlich zu befürchten.

Es ist die Aufgabe der vorliegenden Erfindung, die obigen Probleme zu lösen und die Registrierung von Bildinformationen in einem Navigationssystem auch für Bilder aus digitalen Bildaufnahmegeräten, wie zum Beispiel einem digitalen C-Bogen-Röntgengerät zu ermöglichen. Insbesondere soll ein Verfahren zur Zuordnung einer digitalen Bildinformation zu den Navigationsdaten eines medizinischen Navigationssystems geschaffen werden, welches keine Fehlregistrierungen aufgrund von Zeitverzögerungen bei der Bildinformationsübermittlung mehr befürchten lässt.

Diese Aufgabe wird gemäß einem Aspekt der vorliegenden Erfindung gelöst durch ein Verfahren zur Zuordnung einer digitalen Bildinformation zu den Navigationsdaten eines medizinischen Navigationssystems, bei dem eine digitale Bildinformation aus einem digitalen Bildaufnahmegerät für einen Patienten erstellt wird, der mittels des Navigationssystems überwacht wird, bei der Bilderstellung ein Signal vom Bildaufnahmegerät an das Navigationssystem übersandt wird, welches eine Zuordnungsinformation für die Zuordnung der Bildinformation zu den für diese Bildinformation geltenden Navigationsdaten enthält, die Bildinformation vom Bildaufnahmegerät an das Navigationssystem übersandt wird, und bei dem die Bildinformation und die zugehörigen Navigationsdaten einander zugeordnet werden, wobei das bei der Bilderstellung generierte Signal vom Bildaufnahmegerät selbst generiert wird.

Die Vorteilhaftigkeit der vorliegenden Erfindung beruht insbesondere auf der Übersendung eines Signals (Telegramm) vom Bildaufnahmegerät an das Navigationssystem bei der Bilderstellung. Mit der Übergabe dieses Signals wird dem Navigationssystem mitgeteilt, dass zu einer bestimmten Zeit eine digitale Bildinformation erstellt wird, und da ein solches Signal nicht unbedingt die Bildinformation selbst enthalten muss, kann es ohne Zeitverzögerung vom digitalen Bildaufnahmegerät abgesendet werden. Damit wird die digitale Übergabe von Daten für die Navigation erst ermöglicht, und sie kann zumindest mit der gleichen Genauigkeit durchgeführt werden, wie mit Hilfe des konventionellen Verfahrens, bei dem ständig ein analoges Bild am Navigationssystem anliegt. Die zeitliche Unterbrechung zwischen Aufnahme und Übergabe der Bilddaten vom digitalen Bildaufnahmegerät zum Navigationssystem wird mit Hilfe dieses Signals überbrückt, so dass eine sehr hohe Registrierungsgenauigkeit erreicht werden kann, wobei noch die Vorteile der digitalen Bildinformationen zum Tragen kommen, nämlich die hohe Auflösung und Genauigkeit und die Abspeicherbarkeit der Informationen.

Das vom Bildaufnahmegerät an das Navigationssystem übersandte Signal kann im Rahmen der vorliegenden Erfindung eine sehr unterschiedliche Natur haben. Die dem Signal innewohnende Zuordnungsinformation kann beispielsweise lediglich eine Information sein, die dem Navigationssystem mitteilt, dass eine Bildinformation erstellt worden ist. Es ist also möglich, nur eine Art "Ping"-Signal bei der Bilderstellung vom digitalen Bildaufnahmegerät an das Navigationssystem zu übermitteln, wobei der Empfang des "Ping"-Signals automatisch auslöst, dass das Navigationssystem die zu diesem Zeitpunkt erfassten Navigationsdaten der Bildinformation zuordnet, die erstellt wurde, als das Ping-Signal gesendet wurde. Auch wenn das tatsächliche Bild, also die tatsächliche Bildinformation erst etwas später nach der Verarbeitung am C-Bogen beim Navigationssystem ankommt, ist es deshalb möglich, diesen Bildinformationen die richtigen Navigationsdaten zuzuordnen und damit das Bild korrekt im Navigationssystem zu registrieren.

Bei einer Ausführungsform des erfindungsgemäßen Verfahrens enthält die Zuordnungsinformation eine oder mehrere Informationen über das erstellte Bild, und insbesondere können diese Informationen eine Bildnummer, ein Bilderstellungszeitpunkt sowie andere Informationen über die Bildeigenschaften (zum Beispiel Kalibrierungsinformationen für diese Bildinformation) oder den Patienten beinhalten. Eine digitale Bildinformation wird üblicherweise in Form einer Header-Information plus zugehöriges Bild erstellt und gespeichert. In der Header-Information können dem eigentlichen Bild noch Identifizierungsmerkmale zugeordnet werden, beispielsweise Bildnummer, Bilderstellungszeitpunkt, Auflösung, Bezeichnung des Bildaufnahmegerätes usw. Es ist nun gemäß einer Ausführungsvariante der Erfindung beispielsweise möglich, mit der Signalübersendung bei der Bilderstellung lediglich diese Header-Informationen und ein leeres Bild zu übersenden, da für die Erstellung dieser Header-Informationen praktisch keine Zeit beansprucht wird. Das Bild kann dann nachfolgen und im Navigationssystem den identischen Header-Informationen sowie den für diesen Header erstellten Navigationsdaten zugeordnet werden.

Es ist ferner möglich, in das Signal (Telegramm) noch Referenzinformationen über das Bild aufzunehmen, zum Beispiel über die Lage des Bildes im Raum, was besonders dann vorteilhaft ist, wenn das Bild zur Ansicht softwareseitig gedreht oder verschoben wird. Ebenso können Daten über die Lage des Bildverstärkers oder Darstellungsinformationen (zum Beispiel Kontrast) übermittelt werden.

In seltenen Fällen, bei denen die Verarbeitungszeit am Bildaufnahmegerät für die digitale Bildinformation äußerst gering gehalten werden kann, ist es auch im Rahmen der vorliegenden Erfindung möglich, das gesamte Bild zusammen mit dem Signal und der Zuordnungsinformation gleichzeitig an das Navigationssystem zu übersenden. Dies wäre ein Optimalfall für das Verfahren gemäß der vorliegenden Erfindung, welches aber immer auch die Sicherheit gewährleistet, dass längere Verarbeitungszeiten keine Ungenauigkeiten bei der Registrierung hervorrufen.

Im Allgemeinen wird die digitale Bildinformation aber erst im digitalen Bildaufnahmegerät verarbeitet und gespeichert werden müssen, wobei etwas Zeit vergeht. Deshalb erfasst die vorliegende Erfindung gemäß ihrer bevorzugten Ausführungsform auch diejenigen Fälle, bei denen die Bildinformation zeitversetzt gegenüber der Übersendung des Signals an das Navigationssystem übersandt wird. Vorzugsweise werden nacheinander erstellte Bildinformationen und/oder die zugeordneten Navigationsdaten abrufbar abgespeichert, beispielsweise jeweils im Bildaufnahmegerät und im Navigationssystem oder in einer der beiden Einheiten oder in einem separaten, zentralen Speicher. Wenn also Bilddaten später als das Signal bei der Bilderstellung an das Navigationssystem gesendet und dort empfangen werden, werden die Positionsdaten, die das Navigationssystem bei dem Erhalt des Signals gespeichert hat, den Bilddaten zugeordnet, die ebenfalls die Kodierung des Signals tragen. Durch eine exakte Aufnahme der Position des Bildaufnahmegerätes und des Patienten wird damit eine genaue Registrierung für die Navigation möglich.

Bei einer weiteren Variante wäre es möglich, die Genauigkeit noch dadurch zu erhöhen, dass das Navigationssystem kontinuierlich die Positionsdaten des Bildaufnahmegerätes und des Patienten speichert. Beim Empfang des Signals sowie der Zuordnungsinformation kann dann auf eine frühere Positionskoordinate zurückgerechnet werden. Mit anderen Worten wird die Zeitverzögerung zwischen dem Zeitpunkt der Erstellung der Bildinformation und dem Zeitpunkt der Erfassung der Navigationsdaten vorab erfasst und bei der Zuordnung der Bildinformation zu den Navigationsdaten berücksichtigt bzw. herausgerechnet.

Insbesondere ist ein solches System dann hilfreich, wenn auch das Senden des Signals mit der Zuordnungsinformation merklich Zeit in Anspruch nimmt. Oftmals werden digitale Bildaufnahmegeräte und Navigationssysteme nicht direkt miteinander verbunden, sondern über ein hausinternes Datennetz, was durchaus zu relevanten Verzögerungen bei der Signalübertragung führen kann. In einem solchen Fall ist es insbesondere von Vorteil, wenn gemäß einer Variante des erfindungsgemäßen Verfahrens die Zeitverzögerung zwischen dem Zeitpunkt der Übersendung des Signal und dem Zeitpunkt der Erfassung der Navigationsdaten für ein bestimmtes Datenübertragungssystem vorab erfasst und bei der Zuordnung der Bildinformation zu den Navigationsdaten berücksichtigt bzw. herausgerechnet wird. Dies gilt auch in Fällen der drahtlosen Kommunikation zwischen den Geräten, zum Beispiel in einem drahtlosen Datenübertragungsnetzwerk, wo Verzögerungen beispielsweise aufgrund von Übermittlungsstörungen auftreten können. Auch hierfür eignet sich die vorliegende Erfindung besonders.

Bei einer bevorzugten Ausführungsform der vorliegenden Erfindung beinhaltet die digitale Bildinformation ein Röntgenbild, das von einem C-Bogen-Röntgengerät erstellt wird, welches die Röntgenbilddaten digital verarbeitet und erfasst.

Die vorliegende Erfindung umfasst ferner ein Programm, das, wenn es auf einem Computer läuft oder in einem Computer geladen ist, den Computer veranlasst, das Verfahren durchzuführen, wie es oben in seinen Ausführungsvarianten erläutert worden ist. Ferner umfasst die Erfindung ein Computerprogramm-Speichermedium, welches ein solches Programm aufweist.

Gemäß einem weiteren Aspekt der vorliegenden Erfindung betrifft diese eine Einrichtung zur Zuordnung einer digitalen Bildinformation zu den Navigationsdaten eines medizinischen Navigationssystems, mit einem digitalen Bildaufnahmegerät, das eine digitale Bildinformation für einen Patienten erstellt, der mittels des Navigationssystems überwacht wird, mit einer Signalübertragungseinrichtung, mittels der bei der Bilderstellung ein Signal vom Bildaufnahmegerät an das Navigationssystem übersandt wird, welches eine Zuordnungsinformation für die Zuordnung der Bildinformation zu den für diese Bildinformation geltenden Navigationsdaten enthält, mit einer Bildübertragungseinrichtung, mittels der die Bildinformation vom Bildaufnahmegerät an das Navigationssystem übersandt wird, und mit einer Datenverarbeitungseinrichtung, mittels der die Bildinformation und die zugehörigen Navigationsdaten einander zugeordnet werden, und mit einem Bildaufnahmegerät, welches das Signal bei der Bilderstellung selbst generiert.

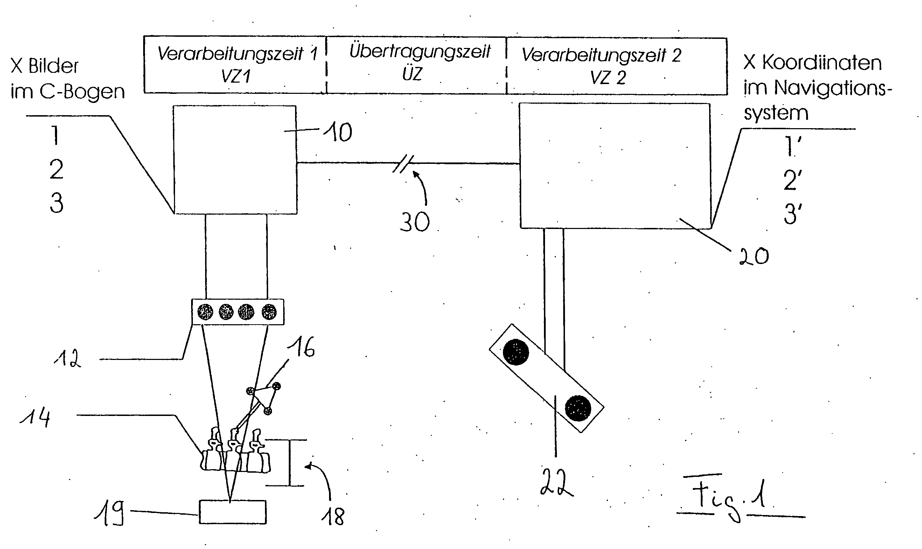

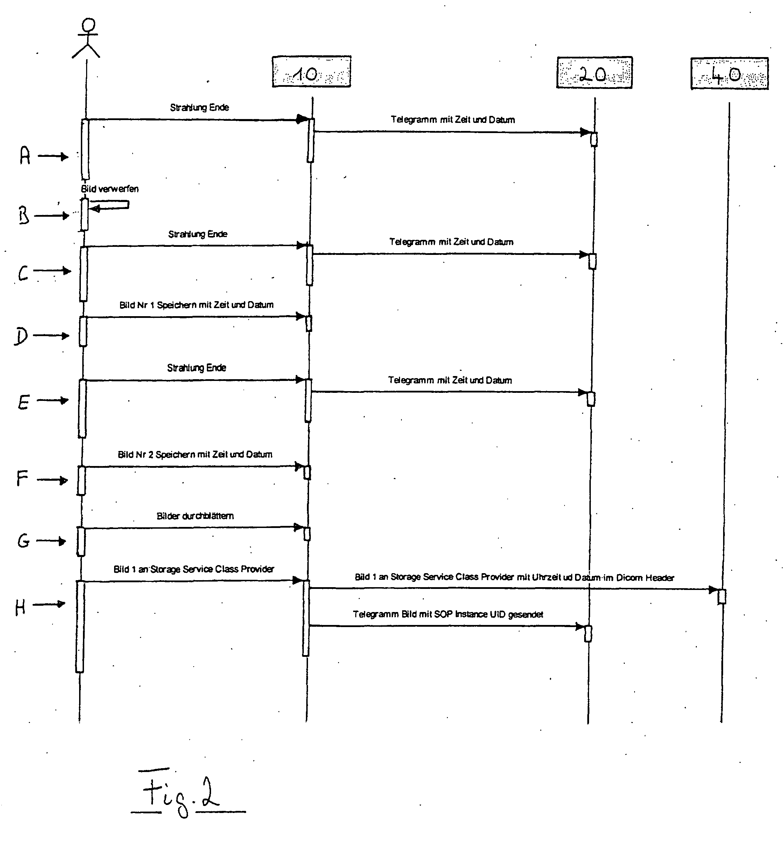

Die Erfindung wird nunmehr mit Hilfe der beiliegenden Zeichnungen und anhand von Ausführungsbeispielen näher erläutert. In der Figur 1 ist die apparative Anordnung und die Signalübertragung bei einem erfindungsgemäßen System skizziert, während die Figur 2 ein Ablauf- bzw. Interaktionsdiagramm für einen erfindungsgemäßen Verfahrensablauf zeigt.

Die wichtigsten Hardwarekomponenten sind in Figur 1 systematisch dargestellt. Es handelt es sich um den C-Bogen 10, an dem eine Referenzeinrichtung 12 befestigt ist, mit der unter anderem seine Position im Navigationssystem bestimmt werden kann. Mittels des C-Bogens 10 werden Röntgenbilder eines Patienten 14 erstellt, der wiederum mittels der Referenzeinrichtung 16 durch das Navigationssystem getrackt, d. h. positionell verfolgt und bestimmt werden kann. Mit dem Bezugszeichen 18 ist angedeutet, dass der Patient sich möglicherweise bewegen kann.

Das Bild des C-Bogens wird auf einem Bildaufnehmer 10 aufgenommen; dabei ist mit dem Bezugszeichen 19 die Röntgenquelle aufgezeigt.

Bei dem C-Bogen 10 handelt es sich um einen digital arbeitenden C-Bogen, der die am Bildaufnehmer 10 ankommenden Informationen zu digitalen Bildern verarbeiten kann und abspeichert. Links am C-Bogen 10 ist dargestellt, dass mit dem vorliegenden System beispielsweise drei Bilder erstellt worden sind, die nacheinander mit den Ziffern 1, 2 und 3 nummeriert werden. In der Praxis wird der Bediener des Systems solange einzelne Bilder erstellen, bis er eines gefunden hat, welches die wichtigen körperlichen Merkmale gerade so darstellt, wie dies für eine Einblendung des Röntgenbildes in das operationsunterstützende Bildmaterial optimal ist. Dabei werden immer wieder die Bildinformationen, die am Bildaufnehmer 19 ankommen, zu digitalen Bildern verarbeitet, und sie können auch schon in einem Speicher des C-Bogens abgespeichert werden, so dass sie später auch wieder abrufbar sind.

Um die Röntgenbilder später bei der Operation richtig in das Bildmaterial des Navigationssystems einblenden zu können, muss die Position des Patienten im Zeitpunkt der Aufnahme des Röntgenbildes bekannt sein, und deshalb wird bei der Röntgenbilderstellung die Position des Patienten 14 über die Referenzeinrichtung 16 erfasst, und zwar mittels des Navigationssystems 20, das die Referenzeinrichtung 16 in Relation zu einer Referenz bzw. Referenzeinrichtung 12 am C-Bogen 10 über ein Kamerasystem 22 erfasst. Es bestünde nun grundsätzlich die Möglichkeit, dem Bediener einen Schalter in die Hand zu geben, der bei Betätigung dem Navigationssystem immer mitteilt, dass gerade in diesem Moment ein Röntgenbild erstellt wird, jedoch ist eine solche Vorgehensweise fehleranfällig, wenn der Auslöser beispielsweise verspätet betätigt wird, und außerdem soll grundsätzlich versucht werden, den Bedienungsaufwand möglichst gering zu halten.

Hier greift nunmehr die vorliegende Erfindung ein, und zwar über die Optimierung der Datenverbindung und Signalübertragung zwischen C-Bogen 10 und Navigationssystem 20. Dargestellt ist eine solche Verbindung als Schnittstellenleitung 30 zwischen C-Bogen 10 und Navigationssystem 20. Über die Leitung 30 können Informationen vom C-Bogen zum Navigationssystem übertragen werden, und diese Leitung 30 kann einerseits ein Kabel sein, welches die beiden Geräte unmittelbar miteinander verbindet; andererseits ist es möglich, die beiden Geräte über ein hausinternes Netzwerk miteinander zu verbinden.

Das grundsätzliche Problem besteht nun in einer Zeitverzögerung zwischen der Aufnahme des Röntgenbildes und der Positionsermittlung durch das Navigationssystem, die zwei Ursachen haben kann. Die eine Ursache liegt in der Verarbeitungszeit VZ1 am C-Bogen. Das Röntgenbild steht nämlich, weil es digital verarbeitet werden muss, oft nicht unmittelbar zum Zeitpunkt der Bilderstellung zur Verfügung, sondern möglicherweise erst später. Wenn in einem solchen Fall dieses Röntgenbild über die Schnittstellenleitung 30 zum Navigationssystem übertragen wird, um die Navigationserfassung, also die Positionsbestimmung des Patienten 14 auszulösen, ist es gut möglich, dass sich der Patient 14 in der verstrichenen Zeit schon etwas bewegt hat, wobei eine solche Bewegung auch nur aus der Atmung resultieren kann. Das Navigationssystem 20 würde dann dem erhaltenen Bild Navigationsdaten zuordnen, die positionell nicht mehr mit denjenigen übereinstimmen, die zum Zeitpunkt der Bilderstellung galten. Aus diesem Grund wird gemäß der vorliegenden Erfindung bei der Bilderstellung ein Signal vom C-Bogen 10 an das Navigationssystem 20 übersandt, welches eine Zuordnungsinformation für die Zuordnung der Bildinformation enthält, wobei ein solches Signal unmittelbar bei Bilderstellung übersandt werden kann, da es nicht unbedingt schon das Bild selbst enthalten muss.

Im dargestellten Beispielsfall handelt es sich bei dem Signal um eines, das als Zuordnungsinformation die Nummer des Bildes, also 1, 2 oder 3 aufweist. Dieses Signal kann praktisch ohne Zeitverzögerung im C-Bogen 10 generiert und über die Schnittstellenleitung 30 zum Navigationssystem 20 übertragen werden. Das Navigationssystem 20 kann dann praktisch zum Bilderstellungszeitpunkt (beispielsweise in dem Fall, bei dem C-Bogen 10 und Navigationssystem 20 direkt per Kabel miteinander verbunden sind) eine Positionserfassung durchführen und dem Röntgenbild 1 die Koordinaten 1', d. h. die zugehörigen Navigationsdaten zuordnen. Wenn dann zu einem späteren Zeitpunkt das Bild 1 aus dem C-Bogen 10 über die Schnittstellenleitung 30 zum Navigationssystem 20 übersandt wird, ist dies unschädlich, da dort schon die vorher erfassten und abgespeicherten Navigationsdaten 1' vorliegen, auch wenn sich der Patient 14 in der Zwischenzeit bewegt hat. Dieser Vorgang kann natürlich für alle erstellten Bilder 1, 2, 3 durchgeführt werden, denen dann die zeitlich richtigen Navigationsdaten bzw. -koordinaten 1', 2', 3' zugeordnet werden.

Die Zuordnungsinformation muss nicht immer eine Bildnummer sein. Sie kann nur aus einem kurzen Auslösesignal, einem sogenannten Ping, bestehen, oder auch mehrere schnell zu generierende Informationen beinhalten, wie beispielsweise Zeit, Datum, Kalibrierungsinformationen, Patienteninformationen u. ä. Wenn die Nummerierung verwendet wird, kann diese beim Neustart beider Systeme immer wieder auf Null gesetzt werden.

Um eine noch größere Sicherheit bei der Zuordnung zu gewährleisten, ist es im Rahmen einer vorteilhaften Ausführungsform der Erfindung möglich, die Zeiterfassung, also die Uhren im C-Bogen und im Navigationssystem zu synchronisieren. Es kann dann in allen Zweifelsfällen auch auf den im Rahmen des Telegramms (Signals) übermittelten, absoluten Zeitpunkt bei der Zuordnung zurückgegriffen werden.

Die vorliegende Erfindung sichert deshalb unter anderem den Fall ab, dass die Bildverarbeitungsdauer am C-Bogen zu Verzögerungen bei der Navigationserfassung führt. Wenn vorab aber sicher feststeht, dass die Bildverarbeitung keinerlei relevante Zeit benötigt, kann auch das Bild selbst zusammen mit dem Signal und der Zuordnungsinformation übertragen werden. Die Erfindung umfasst auch solche Fälle, bietet aber eine Rückversicherung für C-Bögen mit relativ langsamer Bildverarbeitung.

Ein weiteres Problem, das durch eine spezielle Ausführungsform der vorliegenden Erfindung gelöst wird, stellt sich mit der möglicherweise relevanten Übertragungszeit ÜZ zwischen C-Bogen 10 und Navigationssystem 20 (zum Beispiel wenn die beiden über ein hausinternes Datennetz miteinander verbunden sind) und mit der Verarbeitungszeit VZ2 am Navigationssystem. Beide Zeitverzögerungen ÜZ und VZ2 bewirken im schlechtesten Fall noch insgesamt eine Verzögerung bei der Aufnahme der Koordinaten durch das Navigationssystem gegenüber dem Bilderstellungszeitpunkt, jedoch ist auch dieses Problem erfindungsgemäß noch lösbar. Die Übertragungszeit ÜZ und die Verarbeitungszeit VZ2 können sich nämlich vorab feststellen lassen. Wenn das Navigationssystem dann kontinuierlich die Positionsdaten des C-Bogens und des Patienten speichert, kann nach der Verarbeitung des Signals im Navigationssystem einberechnet werden, dass der Bilderstellungszeitpunkt um die Zeitdifferenz ÜZ + VZ2 vorher gelegen haben muss, und es werden dann die für diesen früheren Zeitpunkt gültigen, gespeicherten Navigationsdaten zur Zuordnung der Bildinformationen verwendet. Die zeitliche Unterbrechung durch die Übertragungszeit ÜZ und die Verarbeitungszeit VZ2 am Navigationssystem werden somit "herausgerechnet", und weil vorab schon die Verarbeitungszeit VZ1 mit der erfindungsgemäßen Signalübertragung vor der Bildübertragung "entschärft" wurde, lässt sich gemäß dieser vorteilhaften Ausführungsform eine optimale Genauigkeit bei der Zuordnung der Navigationsdaten und des Röntgenbildes erzielen, und zwar selbst für den Fall, dass ein sehr langsames hausinternes Netzwerk die Verbindung zwischen Navigationssystem und C-Bogen bildet und das Navigationssystem eine merkliche eigene Verarbeitungszeit aufweist.

Die Figur 2 zeigt ein Ablauf- bzw. Interaktionsdiagramm für einen erfindungsgemäßen Verfahrensablauf. Es interagieren, wie oben in Figur 2 dargestellt, ein symbolisch aufgezeigter Benutzer, der C-Bogen 10, das Navigationssystem 20 und ein mit dem Bezugszeichen 40 versehener Server (zum Beispiel ein sogenannter Dicom-Server), nämlich ein Bildspeicher oder Bildarchiv. Der Server 40 kann ein Bildspeicher des Krankenhausnetzwerkes bzw. ein anderer externer Bildspeicher sein, jedoch kann grundsätzlich auch der Bildspeicher des Navigationssystems 20 verwendet werden.

Der Benutzer initiiert die im Folgenden jeweils beschriebenen Vorgänge, die mit den Schritten A bis H in Figur 2 bezeichnet sind. Im ersten Schritt A erzeugt der Benutzer ein Bild am C-Bogen 10, d. h. er initiiert die Strahlung und beendet diese. Zu diesem Zeitpunkt sendet der C-Bogen 10 ein Telegramm an das Navigationssystem 20, also ein Signal, wie es in der bisherigen Beschreibung bezeichnet wurde. Wie ebenfalls schon beschrieben, weist dieses Telegramm nicht das erzeugte Bild selbst auf, welches im C-Bogen 10 erst noch digital verarbeitet werden muss, sondern es umfasst im vorliegenden Ausführungsbeispiel lediglich eine Zeitund eine Datumsinformation. Diese Informationen können sofort erstellt und weitergeschickt werden, so dass hierdurch keine Zeitverzögerung entsteht. Das Navigationssystem 20 speichert nunmehr die Position des C-Bogens 10 über eine dort angeordnete Referenzeinrichtung 12 sowie die Position des Patienten über die Referenzeinrichtung, die in Figur 1 mit dem Bezugszeichen 16 versehen ist, zusammen mit der speziellen Telegramminformation ab.

Im Schritt B stellt der Benutzer bei der Betrachtung des Bildes fest, dass dieses ungeeignet ist, weil es die wichtigen Körperteilinformationen nicht liefert, beispielsweise aufgrund eines schlechten Kontrastes. Er verwirft das Bild, und erstellt im Schritt C ein neues Bild, wobei hierbei wieder die gleichen Vorgänge ablaufen wie beim Schritt A, nämlich Strahlungsinitiierung, Strahlungsende und Telegrammübermittlung.

Im Schritt D stellt der Benutzer fest, dass das Bild die Qualitätskriterien erfüllt und speichert es als Bild Nr. 1 mit zugehöriger Zeit und Datum im C-Bogen-Bildspeicher ab.

Im Schritt E erfolgt nunmehr eine weitere Bildaufnahme mit Telegrammübermittlung wie in den Schritten A und D, und im Schritt F stellt der Benutzer wieder fest, dass das im Schritt E erstellte Bild ebenfalls von guter Qualität ist, und es wird ebenfalls im C-Bogen-Bildspeicher abgespeichert.

Auf diese Weise lassen sich nunmehr eine Vielzahl von Bildern erstellen, und im Schritt G kann der Benutzer die erstellten Bilder auf einer Anzeigevorrichtung (Monitor) durchblättern und sich dasjenige aussuchen, welches seiner Ansicht die optimalste Darstellung liefert. Im vorliegenden Fall ist dies das Bild Nr. 1, und im Schritt H gibt der Benutzer die Anweisung, das Bild Nr. 1 einerseits im Navigationssystem 20 zu registrieren und andererseits an den Bildspeicher 40 zu senden. Dazu wird das Bild Nr. 1 am C-Bogen aus dem Speicher ausgelesen, und die gesamte Bildinformation, also das Bild selbst zusammen mit den Telegrammdaten (Zeit, Datum) werden weiter verschickt. Einerseits erhält das Navigationssystem 20 dann diesen umfassenden Datensatz, und über einen Abgleich der Telegramminformationen, kann nunmehr die Zuordnung des Bildes zu den im Schritt C vom Navigationssystem aufgenommenen Positionsdaten erfolgen, speziell zu der bei der Erstellung des Bildes Nr. 1 vorhandenen Relativposition zwischen Patient und C-Bogen 20. Andererseits wird das Bild auch zusammen mit den Telegramminformationen nochmals in einem separaten Speicher 40 zur Weiterverwendung abgespeichert.

An diesem Ausführungsbeispiel wird deutlich, dass das erfindungsgemäße Verfahren nicht nur die Vermeidung von Ungenauigkeiten aufgrund ungewollter Verzögerungen (Verarbeitungszeiten, Übertragungszeiten) ermöglicht, sondern auch gewollte Verzögerungen zulässt, die beispielsweise dann entstehen, wenn ein Benutzer mehrere Bilder aufnehmen und das Optimale aussuchen möchte.

## Patentansprüche

1. Verfahren zur Zuordnung einer digitalen Bildinformation zu den Navigationsdaten eines medizinischen Navigationssystems (20), bei dem
- eine digitale Bildinformation aus einem digitalen Bildaufnahmegerät (10) für einen Patienten (14) erstellt wird, der mittels des Navigationssystems(20) überwacht wird,
- bei der Bilderstellung ein Signal generiert und an das Navigationssystem (20) übersandt wird, welches eine Zuordnungsinformation für die Zuordnung der Bildinformation zu den für diese Bildinformation geltenden Navigationsdaten enthält,
- die Bildinformation vom Bildaufnahmegerät (10) an das Navigationssystem (20) übersandt wird, und bei dem
- die Bildinformation und die zugehörigen Navigationsdaten einander zugeordnet werden,
**dadurch gekennzeichnet, dass** das bei der Bilderstellung generierte Signal vom Bildaufnahmegerät (10) selbst generiert wird.

2. Verfahren nach Anspruch 1, bei dem die Zuordnungsinformation eine Information ist, die dem Navigationssystem lediglich mitteilt, dass eine Bildinformation erstellt worden ist.

3. Verfahren nach Anspruch 1, bei dem die Zuordnungsinformation eine oder mehrere Informationen über die erstellte Bildinformation beinhaltet.

4. Verfahren nach Anspruch 3, bei dem die Zuordnungsinformation eine oder mehrere der folgenden Informationen enthält:
- eine Bildnummer,
- den Bilderstellungszeitpunkt,
- Informationen über Bildeigenschaften.

5. Verfahren nach einem der Ansprüche 1 bis 4, bei dem die Bildinformation gleichzeitig mit der Übersendung des Signals an das Navigationssystem übersandt wird.

6. Verfahren nach einem der Ansprüche 1 bis 4, bei dem die Bildinformation zeitversetzt gegenüber der Übersendung des Signals an das Navigationssystem übersandt wird.

7. Verfahren nach einem der Ansprüche 1 bis 6, bei dem nacheinander erstellte Bildinformationen und/oder die zugeordneten Navigationsdaten abrufbar abgespeichert werden.

8. Verfahren nach Anspruch 6 oder 7, bei dem die Zeitverzögerung zwischen dem Zeitpunkt der Erstellung der Bildinformation und dem Zeitpunkt der Erfassung der Navigationsdaten für ein bestehendes Datenübertragungssystem vorab erfasst und bei der Zuordnung der Bildinformation zu den Navigationsdaten berücksichtigt bzw. herausgerechnet wird.

9. Verfahren nach Anspruch 6 oder 7, bei dem die Zeitverzögerung zwischen dem Zeitpunkt der Übersendung des Signals und dem Zeitpunkt der Erfassung der Navigationsdaten für ein bestehendes Datenübertragungssystem vorab erfasst und bei der Zuordnung der Bildinformation zu den Navigationsdaten berücksichtigt bzw. herausgerechnet wird.

10. Verfahren nach einem der Ansprüche 1 bis 9, bei dem die digitale Bildinformation ein Röntgenbild oder Röntgenbilder beinhaltet, die von einem C-Bogen-Röntgengerät erstellt werden, welches die Röntgenbilddaten digital verarbeitet und erfasst.

11. Programm, das, wenn es auf einem Computer läuft oder in einem Computer geladen ist, den Computer veranlasst, das Verfahren gemäß einem der Ansprüche 1 bis 10 durchzuführen.

12. Computerprogramm-Speichermedium, welches das Programm nach Anspruch 11 aufweist.

13. Einrichtung zur Zuordnung einer digitalen Bildinformation zu den Navigationsdaten eines medizinischen Navigationssystems (20), mit einem digitalen Bildaufnahmegerät (10), das eine digitale Bildinformation für einen Patienten (14) erstellt, der mittels des Navigationssystems (20) überwacht wird, mit einer Signalübertragungseinrichtung (30), mittels der bei der Bilderstellung ein Signal an das Navigationssystem (20) übersandt wird, welches eine Zuordnungsinformation für die Zuordnung der Bildinformation zu den für diese Bildinformation geltenden Navigationsdaten enthält, mit einer Bildübertragungseinrichtung (30), mittels der die Bildinformation vom Bildaufnahmegerät (10) an das Navigationssystem (20) übersandt wird, und mit einer Datenverarbeitungseinrichtung, mittels der die Bildinformation und die zugehörigen Navigationsdaten einander zugeordnet werden, **gekennzeichnet durch** ein Bildaufnahmegerät (10), welches das Signal bei der Bilderstellung selbst generiert.

## Claims

1. A method for assigning digital image information to the navigational data of a medical navigation system, wherein:
- digital image information from a digital image recording device (10) is produced for a patient (14) being monitored by means of said navigation system (20);
- a signal is generated and transmitted to said navigation system (20) when an image is produced, said signal including assignment information for assigning said image information to said navigational data which apply to said image information;
- said image information is transmitted from said image recording device (10) to said navigation system (20); and wherein
- said image information and said corresponding navigational data are assigned to each other
**characterised in that** the signal generated when an image is produced is generated by the image recording device (10) itself.

2. The method as set forth in claim 1, wherein said assignment information is information which merely informs said navigation system that image information has been produced.

3. The method as set forth in claim 1, wherein said assignment information includes one or more items of information about said image information produced.

4. The method as set forth in claim 3, wherein said assignment information includes one or more of the following items of information:
- an image number;
- a time of image production;
- information on properties of said image.

5. The method as set forth in any one of claims 1 to 4, wherein said image information is transmitted as the same time as said signal is transmitted to said navigation system.

6. The method as set forth in any one of claims 1 to 4, wherein said image information is transmitted deferred with respect to transmitting said signal to said navigation system.

7. The method as set forth in any one of claims 1 to 6, wherein successively produced image information and/or said assigned navigational data are retrievably stored.

8. The method as set forth in claim 6 or 7, wherein the time delay between the time of producing said image information and the time of capturing said navigational data for an existing data transmission system is determined beforehand and is taken into account or calculated out when assigning said image information to said navigational data.

9. The method as set forth in claim 6 or 7, wherein the time delay between the time of transmitting said signal and the time of capturing said navigational data for an existing data transmission system is determined beforehand and taken into account or calculated out when assigning said image information to said navigational data.

10. The method as set forth in claim 1 or 7, wherein said digital image information includes an x-ray image or x-ray images produced by a C-arc x-ray device which digitally captures and processes said x-ray image data.

11. A program which, when it is running on a computer or is loaded on a computer, causes said computer to perform the method in accordance with any one of claims 1 to 10.

12. A computer program storage medium comprising the program as set forth in claim 11.

13. A means for assigning digital image information to the navigational data of a medical navigation system (20), comprising: a digital image recording device (10) which produces digital image information for a patient (14) being monitored by means of said navigation system (20); a signal transmission means (30) by means of which a signal is transmitted to said navigation system (20) when an image is produced, said signal including assignment information for assigning said image information to said navigational data which apply to said image information; an image transmission means (30) by means of which said image information is transmitted from said image recording device (10) to said navigation system (20); and a data processing means by means of which said image information and said corresponding navigation data are assigned to each other, **characterised by** an image recording device (10) which generates the signal itself when an image is produced.

## Revendications

1. Procédé d'allocation d'information d'image numérique aux données de navigation d'un système de navigation pour applications médicales (20), dans lequel
- une information d'image numérique est réalisée avec un appareil numérique de prise de vues (10) pour un patient (14) qui est surveillé à l'aide du système de navigation (20),
- un signal est généré lors de la réalisation de l'image et envoyé au système de navigation (20), lequel contient une information d'affectation pour l'affectation de l'information d'image aux données de navigation valables pour cette information d'image,
- l'information d'image de l'appareil de prise de vues (10) est transmise au système de navigation (20), et dans lequel
- l'information d'image et les données de navigation correspondantes sont affectées l'une à l'autre,
**caractérisé en ce que** le signal généré lors de la réalisation de l'image est généré par l'appareil de prise de vues (10) lui-même.

2. Procédé suivant la revendication 1, pour lequel l'information d'affectation est une information qui communique simplement au système de navigation qu'une information d'image a été réalisée.

3. Procédé suivant la revendication 1, pour lequel l'information d'affectation contient une ou plusieurs informations concernant l'information d'image réalisée.

4. Procédé suivant la revendication 3, pour lequel l'information d'affectation contient une ou plusieurs des informations suivantes:
- un numéro d'image,
- le moment de la réalisation de l'image,
- des informations sur les caractéristiques de l'image.

5. Procédé suivant l'une des revendications 1 à 4, pour lequel l'information d'image est envoyée au système de navigation en même temps que la transmission du signal.

6. Procédé suivant l'une des revendications 1 à 4, pour lequel l'information d'image est envoyée au système de navigation décalée dans le temps par rapport à la transmission du signal.

7. Procédé suivant l'une des revendications 1 à 6, pour lequel des informations d'image réalisées successivement et/ou les données de navigation correspondantes sont mémorisées de manière permettant de les rappeler.

8. Procédé suivant la revendication 6 ou 7, pour lequel le retard entre le moment de la réalisation de l'information d'image et le moment de l'acquisition des données de navigation est mesuré au préalable pour un système transmission de données existant et pris en compte ou calculé lors de l'affectation de l'information d'image aux données de navigation.

9. Procédé suivant la revendication 6 ou 7, pour lequel le retard entre le moment de la transmission du signal et le moment de l'acquisition des données de navigation est mesuré au préalable pour un système transmission de données existant et pris en compte ou calculé lors de l'affectation de l'information d'image aux données de navigation.

10. Procédé suivant l'une des revendicatio ns 1 à 9, pour lequel l'information d'image numérique comprend une ou plusieurs images radiographiques qui ont été réalisées par un appareil à rayons X à courbure en C, lequel traite et saisit de manière numérique les données des images radiographiques.

11. Programme qui, lorsqu'il tourne sur un ordinateur ou est chargé dans un ordinateur, amène l'ordinateur à exécuter un procédé suivant l'une des revendications 1 à 10.

12. Support d'information pour programme d'ordinateur qui présente le programme suivant la revendication 11.

13. Dispositif d'affectation d'une information d'image numérique aux données de navigation d'un système de navigation pour applications médicales (20), avec un appareil numérique de prise de vues (10), qui réalise une information d'image numérique pour un patient (14) qui est surveillé à l'aide du système de navigation (20), avec un dispositif de transmission de signal (30) avec lequel un signal est envoyé au système de navigation (20) lors de la réalisation de l'image, lequel contient une information d'affectation pour l'affectation de l'information d'image aux données de navigation valables pour cette information d'image, avec un dispositif de transmission d'image (30) avec lequel l'information d'image de l'appareil de prise de vues (10) est envoyée au système de navigation (20) et avec un dispositif de traitement des données avec lequel l'information d'image et les données de navigation correspondantes sont affectées l'une à l'autre, **caractérisé par** un appareil de prise de vues (10) qui génère lui-même le signal lors de la réalisation de l'image.
